(19) Europäisches Patentamt
European Patent Office
Office européen des brevets

(11) EP 4 534 009 A1

(12) **EUROPEAN PATENT APPLICATION**
published in accordance with Art. 153(4) EPC

(43) Date of publication:
**09.04.2025 Bulletin 2025/15**

(21) Application number: **23811926.7**

(22) Date of filing: **09.01.2023**

(51) International Patent Classification (IPC):
***A61B 5/1495** (2006.01)* ***A61B 5/145** (2006.01)*

(52) Cooperative Patent Classification (CPC):
**A61B 5/145; A61B 5/1495**

(86) International application number:
**PCT/KR2023/000398**

(87) International publication number:
**WO 2023/229137 (30.11.2023 Gazette 2023/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **26.05.2022 KR 20220064998**

(71) Applicant: **i-Sens, Inc.**
**Seoul 06646 (KR)**

(72) Inventors:
- **NAH, Ji Seon**
  **Seoul 06646 (KR)**
- **SEO, Jung Hee**
  **Seoul 06646 (KR)**
- **JANG, Min Ji**
  **Seoul 06646 (KR)**
- **KANG, Yun Hee**
  **Seoul 06646 (KR)**

(74) Representative: **BCKIP Part mbB**
**MK1**
**Landsbergerstraße 98, 3.Stock**
**80339 München (DE)**

(54) **METHOD FOR CORRECTING BIOMETRIC VALUE, ACCORDING TO WHETHER BIOMETRIC VALUE IS STABILIZED, IN BIOMETRIC INFORMATION MEASUREMENT SYSTEM**

(57) The present invention relates to a method for correcting a biometric value of a user in a biometric information measurement system and, more specifically, to a biometric value correction method, which determines, from the difference between a blood biometric value and a body fluid biometric value, whether a biometric value is stabilized when a correction factor is calculated using a reference biometric value, so as not to use the correction factor calculated from the reference biometric value if the biometric value is unstable and to use a revised correction factor obtained by weighting the correction factor, and thus can accurately determine a corrected biometric value of a user from a biometric value measured by a sensor.

FIG. 5

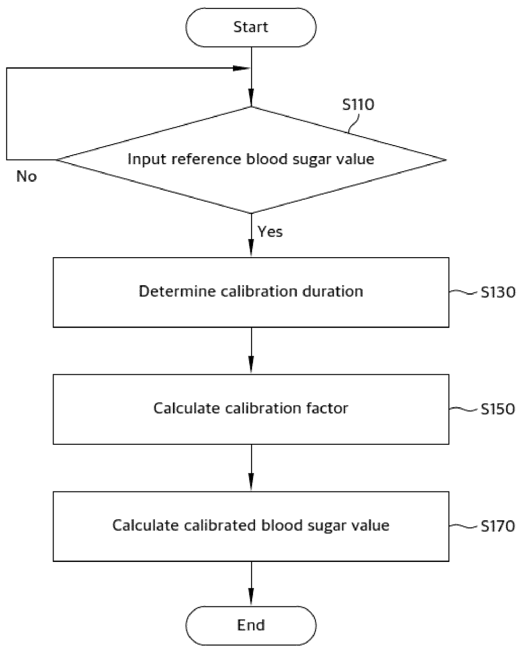


EP 4 534 009 A1

## Description

### Technical Field

The present disclosure relates to a method for calibrating a biometric value of a user in biometric information measurement system, more specifically, to a calibration method for a biometric value, in which when a calibration factor is calculated by using a reference biometric value, whether the biometric value is stabilized may be determined from a difference between a blood biometric value and an interstitial fluid biometric value, and when the biometric value is unstable, a calibrated biometric value of a user may be accurately determined from a biometric value measured in a sensor by using a modified calibration factor with a weight value granted to the calibration factor, not using the calibration factor calculated from the reference biometric value in an intact manner.

### Background Art

Diabetes is a chronic disease that largely occurs among contemporary people. In the Republic of Korea, two million or more people that are 5% of domestic population have diabetes.

Diabetes occurs when an absolutely large amount of a sugar component is in blood because a balance between the blood and sugar is not redressed as insulin formed in pancreas is absolutely or relatively insufficient due to various causes such as obesity, stress, a poor diet, and innate heredity.

Generally, the blood contains a specific concentration of glucose, and a tissue cell gains energy therefrom.

However, when increased more than necessary, the glucose is not appropriately stored in a muscle or a fat cell and is accumulated in the blood. Accordingly, a diabetes patient maintains blood sugar greatly higher than a normal person. As the superabundant blood sugar directly passes through tissues to be discharged in urine, a sugar component absolutely required in each of the tissues of a body becomes insufficient, which causes an abnormality in each of the tissues of the body.

Diabetes has a feature that a subjective symptom thereof is not approximately obvious at an initial stage. As diabetes progresses, unique symptoms of diabetes, such as eating much, drinking much, polyuria, weight loss, general malaise, itching, and hand and foot injuries that are not cured and last long, appear. When diabetes further progresses, complications that develop into visual disturbance, high blood pressure, kidney disease, stroke, periodontal disease, a cramp, neuralgia, gangrene, and the like appear.

In order to diagnose diabetes and manage diabetes to prevent developing the complications, systematic measurement and treatment of the blood sugar may be done simultaneously.

Since diabetes requires constant measurement of blood sugar, demand for a device associated with blood sugar measurement is constantly increased. Various researches show that occurrence of the complications of diabetes is greatly reduced when the diabetes patient strictly controls the blood sugar. Accordingly, regularly measuring the blood sugar is greatly important for the diabetes patient.

In order to control the blood sugar of the diabetes patient, such a blood gathering-type blood sugar meter that uses a finger prick method is mainly used. Such a blood gathering-type blood sugar meter helps control of the blood sugar of the diabetes patient, but accurately identifying a blood sugar value that frequently changes is difficult because the blood gathering-type blood sugar meter shows only a result at a time of measuring. In addition, Since the blood gathering-type blood sugar meter always requires blood gathering in order to frequently measure the blood sugar in one day, the diabetes patient has a large burden of blood gathering.

In order to overcome the limitation on such a blood gathering-type blood sugar meter, a continuous glucose monitoring system (CGMS) that is inserted into a human body to measure the blood sugar at intervals of several minutes has been developed. Control for the diabetes patient and coping with the emergency may be facilitated with the continuous glucose monitoring system.

The continuous glucose monitoring system includes and is formed of a sensor transmitter that is attached to a body of a user and measures a biometric value from an interstitial fluid (ISF), a communication terminal that outputs information on the received biometric value to the user, or the like. The sensor transmitter includes a sensor for continuous blood sugar measurement, of which a portion is inserted into a human body. The sensor is inserted into the human body for a predetermined use period, for example, fifteen days and continuously measures the biometric value. The sensor transmitter periodically measures the biometric value from the interstitial fluid. A biometric management application is installed in the communication terminal, and the communication terminal periodically receives the biometric value from the sensor transmitter and outputs the information on the received biometric value to the user.

The sensor of the sensor transmitter is continuously inserted into skin for the use period. Sensor sensitivity may be different depending on a body region into which the sensor is inserted. Although a sensor insertion position on the body region is identical, the sensitivity of the sensor is changed as time elapses. The biometric value measured in the sensor transmitter has an error in an actual biometric value of the user depending on a change in the sensitivity. In order to overcome such an error, the biometric value of the user may be calibrated by applying a calibration factor to the measure biometric value.

In order to accurately provide an accurate biometric value, the biometric value received from the sensor transmitter may be initially calibrated and then continuously calibrated at predetermined calibration periods in a use period of the sensor transmitter. More specifically, the calibration factor may be continuously calculated, with a reference biometric value measured through an additional measurement device and the biometric value received from the sensor transmitter, at the calibration periods in the use period of the sensor transmitter, and the biometric value received from the sensor transmitter may be calibrated by applying the calibration factor before a next calibration period arrives.

The biometric value which is calibrated as such is an interstitial fluid biometric value measured from the interstitial fluid of the user. There is a time delay between the interstitial fluid biometric value and a blood biometric value measured from blood or plasma.

FIG. 1 is a diagram for describing a delay model between an interstitial fluid biometric value and a blood biometric value.

As illustrated in FIG. 1, a blood vessel and an interstitial fluid are separated by a barrier of a capillary, and a time delay occurs when glucose diffuses from the blood vessel to the interstitial fluid by diffusion.

An interstitial fluid blood sugar value $I_{glu}$ is calculated as shown in Equation 1.

[Equation 1]

$$I_{glu} = I_{sig} \times \alpha + b$$

Here, $I_{sig}$ denotes a sensor signal showing a biometric value, which is measured in a sensor and is a general electric current value. $\alpha$ denotes a calibration factor, and b denotes an offset.

A final blood biometric value is calculated by using the delay model between the interstitial fluid biometric value and the blood biometric value, such as Equation 2, and a state transition matrix of a Kalman filter such as Equation 3.

[Equation 2]

$$\frac{dB}{dt} = -\frac{1}{\tau} B + \frac{1}{\tau} I_{glu}$$

Here, B denotes the final blood biometric value, and $\tau$ denotes the time delay.

[Equation 3]

$$I_{k+1} = \phi I_k + \Gamma B_k, \phi = e^{\frac{\triangle t}{\tau}}, \Gamma = 1 - \phi$$

In addition to such a time delay according to the delay model between the interstitial fluid biometric value and the blood biometric value, a time delay such as a delay due to a physical structure of the sensor itself or a calculation delay required for calculating the interstitial fluid biometric value from the sensor signal occurs.

Thus, the calibration factor is calculated from a reference biometric value and the sensor signal which is temporally delayed, for example, temporally delayed for one minute to fifteen minutes from a time point of measuring the reference biometric value. When the calibration factor is calculated, the reference biometric value and the sensor signal which is mapped to the reference biometric value do not have a large difference in a state in which the biometric value is stabilized. However, time delays of the sensor signal may be different from each other depending on a type of the sensor or a user, and since the temporally delayed sensor signal which is mapped to the reference biometric value in a state in which the biometric value is not stabilized is not stabilized, the calibration factor may not be accurately calculated by using the reference biometric value which is measured in the state in which the biometric value is not stabilized.

Meanwhile, a predetermined time is required until the sensor is stabilized after the sensor is inserted into a body. Reliability of the measured biometric value is relatively low before the sensor is stabilized. As the sensor then becomes stabilized, the reliability of the biometric value measured in the sensor is relatively increased. Thus, when the calibration factor is calculated with the reference biometric value measured while the biometric value of the user is unstable, the calibration factor is required to be differently calculated in consideration of a degree of stabilization of the sensor.

## Detailed Description of the Invention

### Technical Goals

The present disclosure is to solve the above-described difficulty of a calculation method for a calibration factor in the related art. The present disclosure is to provide a method of determining whether a biometric value is stabilized from a difference between a blood biometric value and an interstitial fluid biometric value and accurately calculating a calibration factor depending on whether the biometric value of a user is unstable.

The present disclosure is to also provide a method of dividing an entire use period of a sensor into multiple calibration durations and accurately calculating the calibration factor in consideration of a degree of stabilization of the sensor according to the calibration durations when the calibration factor is calculated by using a reference biometric value.

### Technical solutions

According to an aspect, there is provided a calibration method for a biometric signal, the calibration method including determining whether a reference biometric value is input, calculating a first biometric parameter and a second biometric parameter at a time point at which the reference biometric value is input, determining whether a modification condition is satisfied based on the first biometric parameter and the second biometric parameter, and calculating, with calculating a modified calibration factor depending on whether the modification condition is satisfied, a calibrated biometric value from a biometric signal measured in a sensor by using the modified calibration factor.

When the modification condition is not satisfied, a current calibration factor may be calculated from the input reference biometric value, and the calibrated biometric value may be calculated from the biometric signal by using the current calibration factor before a next reference biometric value is input.

Calculating of a blood calibrated biometric value may include, when the modification condition is satisfied, calculating a current calibration factor from the input reference biometric value, determining a calibration duration to which the time point at which the reference biometric value is input belongs, differently calculating the modified calibration factor from the current calibration factor based on the determined calibration duration depending on the calibration duration to which the time point at which the reference biometric value input belongs, calculating an interstitial fluid calibrated biometric value in an interstitial fluid by using the biometric signal measured in the sensor and the modified calibration factor before a next reference biometric value is input, and calculating a blood calibrated biometric value by applying the interstitial fluid calibrated biometric value to a conversion model.

The calibration duration may be divided into a first duration, a second duration after the first duration, and a third duration after the second duration according to a lapse of time from a time point of inserting the sensor into a body.

When the time point at which the reference biometric value is input belongs to the first duration, the modified calibration factor may be the current calibration factor.

When the time point at which the reference biometric value is input belongs to the second duration, the modified calibration factor may be calculated by applying a first weight value to the current calibration factor.

When the time point at which the reference biometric value is input belongs to the third duration, the modified calibration factor may be calculated by applying a second weight value to the current calibration factor.

The first biometric parameter may be an interstitial fluid (ISF) blood sugar value, and the second biometric parameter may be a blood or plasma blood sugar value.

The calibration method may further include calculating an average value of previous modified calibration factors calculated in the third duration from the time point at which the reference biometric value is input, and remodifying the modified calibration factor based on the average value.

The remodifying of the modified calibration factor may include calculating a modification threshold range with the average value as a reference, determining whether the modified calibration factor is out of the modification threshold range, and determining a final calibration factor by remodifying the modified calibration factor depending on whether the modified calibration factor is out of the modification threshold range, and the calibrated biometric value may be calculated by using the final calibration factor.

When the modified calibration factor is out of the modification threshold range, the final calibration factor may be determined by remodifying the modified calibration factor to be in the modification threshold range.

When the modified calibration factor is within the modification threshold range, the modified calibration factor may be determined to be the final calibration factor.

The remodifying of the modified calibration factor may include calculating a difference between the modified calibration factor and the average value, calculating a reward value from the difference between the modified calibration factor and the average value, and calculating a final calibration factor by remodifying the modified calibration factor with the reward value, and the calibrated biometric value may be calculated by using the final calibration factor.

## Effects of the Invention

According to example embodiments, when a calibration factor is calculated by using a reference biometric value, whether a biometric value is stabilized may be determined from a difference between a blood biometric value and an interstitial fluid biometric value, and when the biometric value is unstable, a weight value may be granted to the calibration factor without using the calibration factor calculated from the reference biometric value in an intact manner. Through this, the biometric value of a user may be accurately determined from a biometric signal.

According to example embodiments, when the calibration factor is calculated by using the reference biometric value, an entire use period of a sensor may be divided into multiple calibration durations, and the calibration factor may be modified in consideration of a degree of stabilization of the sensor according to a calibration duration to which a time point at which the reference biometric value is input belongs. Through this, the biometric value of the user may be accurately calculated from the biometric signal of the sensor.

According to example embodiments, an average value of calibration factors in a duration in which the sensor is stabilized may be calculated, and the calibration factor may be remodified in consideration of a difference between a modified calibration factor and the average value. Through this, the biometric value of the user may be accurately calculated from the biometric signal of the sensor in the duration in which the sensor is stabilized.

## Brief Description of Drawings

FIG. 1 is a diagram for describing a delay model between a interstitial fluid biometric value and a blood biometric value.
FIG. 2 is a schematic diagram illustrating a biometric value measurement system according to an example embodiment of the present disclosure.
FIG. 3 is a function block diagram for describing a calibration device for a biometric signal according to the present disclosure.
FIG. 4 is a function block diagram for describing a calibration part according to the present disclosure.
FIG. 5 is a flowchart for describing a calibration method for a biometric signal according to the present disclosure.
FIG. 6 is a diagram for describing an example of inputting calibration information according to a calibration period.
FIG. 7 illustrates an example of a calibration duration dividing an entire use period of a sensor according to the present disclosure.
FIG. 8 is a flowchart for describing an example of calculating a calibration factor in the present disclosure.
FIG. 9 is a flowchart for describing an example of an operation of remodifying a modified calibration factor in the present disclosure.
FIG. 10 is a flowchart for describing another example of an operation of remodifying a modified calibration factor in the present disclosure.

## Mode for Carrying Out the Invention

Technical terms used in the present disclosure is merely to describe predetermined example embodiments. It should be noted that the technical terms is not to limit the present disclosure. Also, the technical terms used in the present disclosure should be construed, unless the terms are specially defined to have other meanings, as having meanings generally understood by those skilled in the art to which the present disclosure belong and should not be construed as having excessively inclusive meanings and excessively narrow meanings. In addition, when being wrong technical terms not accurately describing the idea of the present disclosure, the technical terms used in the present disclosure is to be substituted with technical terms understandable by those skilled in the art.

Also, terms in a singular form used in the present disclosure include terms in a plural form unless an apparently and contextually conflicting description is present. In the present disclosure, terms such as "including" or "comprising" should not be construed as that various elements or various operations described in the present disclosure are necessarily included. The terms should be construed as that some of the elements or the operations may not be included or that additional elements or operations may be further included.

Furthermore, the accompanying drawings are merely to easily understand the idea of the present disclosure. It should be noted that the idea of the present disclosure is not to be construed as being limited by the accompanying drawings.

Hereinafter, a method of calibrating a biometric signal depending on a degree of stabilization of a biometric value according to the present disclosure will be described in detail with reference to the accompanying drawings.

FIG. 2 is a schematic diagram illustrating a biometric value measurement system according to an example embodiment of the present disclosure.

Hereinafter, a blood sugar value will be described as an example of a biometric value, and a reference blood sugar value will be described as an example of a reference biometric value. However, various biometric values other than the blood sugar value may be measured depending on a field to which the present disclosure is applied.

Referring to FIG. 2, a biometric value measurement system 1 according to an example embodiment of the present disclosure includes a sensor transmitter 10 and a communication terminal 30.

The sensor transmitter 10 is attached to a body. When the sensor transmitter 10 is attached to the body, an end of a sensor of the sensor transmitter 10 is inserted into skin to measure, from an interstitial fluid of a human body, a biometric signal periodically showing the blood sugar value, namely, a blood sugar signal.

The communication terminal 30 is a terminal that receives the blood sugar signal from the sensor transmitter 10, calibrates the received blood sugar signal with a calibration factor, perform unit conversion into an interstitial fluid blood sugar value, and then display, to a user, a blood blood sugar value by calculating the blood blood sugar value from the interstitial fluid blood sugar value by using a delay model between the interstitial fluid blood sugar value and the blood blood sugar value. A terminal that may communicate with the sensor transmitter 10, such as a smartphone, a tablet personal computer (PC), or a laptop, may be used as the communication terminal 30. Also, the communication terminal 30 is not limited thereto. Any terminal may be used as far as the terminal includes a communication function and a program or an application may be installed in the terminal.

A blood sugar value obtained by applying the calibration factor to and performing the unit conversion for the blood sugar signal measured by the sensor transmitter 10 is referred to as the interstitial fluid blood sugar value. A blood sugar value calculated from the interstitial fluid blood sugar value by using the delay model between the interstitial fluid blood sugar value and the blood blood sugar value is referred to as the blood blood sugar value. Meanwhile, all of the interstitial fluid blood sugar value and the blood blood sugar value which are calculated by applying the calibration factor to the blood sugar signal are referred to as a calibrated blood sugar value. Meanwhile, the blood sugar signal received from the sensor transmitter is referred to as a measured blood sugar value.

The sensor transmitter 10 transmits the blood sugar signal to the communication terminal 30 by a request from the communication terminal 30 or periodically at each set time. For data communication between the sensor transmitter 10 and the communication terminal 30, the sensor transmitter 10 and the communication terminal 30 may be connected in wired communication with each other by a universal serial bus (USB) cable or the like or may be connected in communication with each other with a wireless communication scheme such as infrared communication, near field communication (NFC), or Bluetooth.

When communication is connected between the sensor transmitter 10 and the communication terminal 30, after initial stabilization of the sensor transmitter 10, an initial calibration factor is calculated by using a reference blood sugar value measured through an additional blood sugar measurement device (not illustrated), and initial calibration for the blood sugar signal is performed by using the initial calibration factor. Afterwards, the communication terminal 30 outputs and provides, to the user, the calibrated blood sugar value by calibrating, with the initial calibration factor, the blood sugar signal received from the sensor transmitter 10.

Here, with respect to the initial stabilization, the sensor is determined as being initially stabilized after a predetermined time, for example, after two hours elapse since the sensor is inserted into the body. Alternatively, the initial stabilization may be determined based on the blood sugar signal. Here, the initial stabilization may be a minimum stabilization time or condition required for immediately notifying the user of the calibrated blood sugar value after the sensor is inserted into the body. An additional time, for example, one to ten days may be required for actually completing stabilization of the sensor.

In order to accurately calibrate the blood sugar signal in the sensor transmitter 10 after the initial stabilization, the communication terminal 30 calculates a new calibration factor by using the reference blood sugar value measured through the additional blood sugar measurement device and calibrates, by using the new calibration factor, the blood sugar signal received from the sensor transmitter at calibration periods in a use period of the sensor transmitter 10.

Desirably, a calibration period may be changed depending on a degree of progress of the stabilization. For example, calibration may be performed at intervals of twelve hours after the initial stabilization, and the calibration may be then performed at intervals of twenty-four hours or forty-eight hours depending on the degree of the process of the stabilization.

FIG. 3 is a function block diagram for describing a calibration device for a blood sugar signal according to the present disclosure.

The calibration device for the blood sugar signal may be implemented in a communication terminal such as a smart terminal that notifies a user of a calibrated blood sugar value while performing communication with a sensor transmitter or may be implemented through an additional reception device.

In specific description with reference to FIG. 3, a calibration notification part 130 provides a calibration notification to the user through a user interface part 110 when a calibration period arrives according to a calibration period stored in a storage part 150.

Desirably, when the calibration period arrives, the calibration notification part 130 may extract a biometric parameter stored in the storage part 150 and compare the extracted biometric parameter and a calibration restriction condition. When the calibration restriction condition is satisfied, the calibration notification part 130 may not provide the calibration notification to the user interface part 110 or may inactivate a calibration icon with which a calibration order is input to the user interface part 110. Here, the biometric parameter may be an interstitial fluid blood sugar value, a blood blood sugar value, a change rate of a blood sugar value, a change speed of the blood sugar value, or the like. The calibration restriction condition may be whether the blood sugar value exceeds a threshold restriction blood sugar value or whether a change rate of the calibrated blood sugar value exceeds a threshold restriction change rate.

When a reference blood sugar value is input through the user interface part 110 as the calibration restriction condition is not satisfied, a calibration part 170 determines a calibration duration to which a time point at which the reference blood sugar value is input belongs or determines whether a modification condition of a calibration factor is satisfied based on a biometric parameter at the time point at which the reference blood sugar value is input. The calibration part 170 calculates the calibration factor based on whether the modification condition of the calibration factor is satisfied and the calibration duration to which the time point at which the reference blood sugar value is input belongs and calculates the calibrated blood sugar value by using the calculated calibration factor.

When the reference blood sugar value is input for calculating the calibration factor, the calibration part 170 determines a measured blood sugar value corresponding to a time of inputting the reference blood sugar value among measured blood sugar values received through a transmission and reception part 190 and generates a calibration pair formed of the reference blood sugar value and the corresponding measured blood sugar value. The calibration part 170 calculates the calibration factor by using the calibration pair. Depending on a field to which the present disclosure is applied, the calibration part 170 may calculate the calibration factor by using a current calibration pair formed of the input reference blood sugar value and the measured blood sugar value, but the calibration part 170 may calculate the calibration factor with a regressive scheme by using the current calibration pair and a past calibration pair in order to calculate an accurate calibration factor.

The calibration part 170 may use, in an intact manner, the calibration factor calculated based on whether the modification condition of the calibration factor is satisfied at the time point at which the reference blood sugar value is input and the calibration duration to which the time point at which the reference blood sugar value is input belong or may modify the calculated calibration factor.

The calibration part 170 calculates the calibrated blood sugar value by calibrating, with the calibration factor, the measured blood sugar value which is received after the reference blood sugar value is input and provides the calculated calibrated blood sugar value to the user through the user interface part 110.

FIG. 4 is a function block diagram for describing a calibration part according to the present disclosure.

In specific description with reference to FIG. 4, when a reference blood sugar value is input, a calibration duration determination part 173 determines a calibration duration to which a time point at which the reference blood sugar value is input based on the time point at which the reference blood sugar value is input.

Meanwhile, a calibration condition determination part 175 determines a first biometric parameter and a second biometric parameter at the time point at which the reference blood sugar value is input and determines whether a modification condition of a calibration factor is satisfied from the first biometric parameter and the second biometric parameter. A calibration factor calculation part 177 calculates a current calibration factor from a current calibration pair or a past calibration pair.

A calibration factor determination part 171 determines the current calibration factor, which is calculated in the calibration factor calculation part 177 based on the calibration duration to which the time point at which the reference blood sugar value is input belongs and whether the modification condition of the calibration factor is satisfied from the first biometric parameter and the second biometric parameter, to be a calibration factor in an intact manner or determines a modified calibration factor, to which the current calibration factor is changed by allowing a calibration factor modification part 178 to modify the current calibration factor, to be the calibration factor.

When the modification condition of the calibration condition is satisfied at the time point at which the reference blood sugar value is input, the calibration factor determination part 171 requests the calibration factor modification part 178 to modify the current calibration factor. When receiving a request for modifying the current calibration factor, the calibration factor modification part 178 calculates the modified calibration factor by granting a weight value to the current calibration factor depending on the calibration duration to which the time point at which the reference blood sugar value is input belongs.

When a degree of stabilization of a sensor is high, depending on the degree of the progress of the stabilization, the calibration factor is required to be maintained so as not to be largely changed. When the time point at which the reference blood sugar value is input is determined as a calibration duration in which the degree of the stabilization of the sensor is high, the calibration factor modification part 178 may remodify the modified calibration factor based on an average value of previous calibration factors in the calibration duration in which the degree of the stabilization of the sensor is high, which are calculated in an average value calculation part 179.

As such, the calibration factor determination part 171 uses, based on whether the modification condition of the calibration factor is satisfied at the time point at which the reference blood sugar value is input, the current calibration factor in an intact manner when the modification condition is not satisfied. However, when the modification condition of the calibration factor is satisfied at the time point at which the reference blood sugar value is input, the calibration factor may be modified by granting different weight values to the current calibration factor depending on the calibration duration to which the time point at which the reference blood sugar value is input belongs, or the modified calibration factor may be remodified based on the average value of the previous calibration factors in the calibration duration in which the degree of the stabilization of the sensor is high.

FIG. 5 is a flowchart for describing a calibration method for a biometric signal according to the present disclosure.

In specific description with reference to FIG. 5, in operation S110, whether a reference blood sugar value is input as a calibration period arrives or by a request from a user irrespective of the calibration period is determined.

In operation S130, when the reference blood sugar value is input, a calibration duration to which a time point at which the reference blood sugar value is input belongs in an entire use period of a sensor is determined. Here, with respect to the calibration duration, the entire use period of the sensor may be divided into a first duration, a second duration, and a third duration according to a lapse of time from a time point of inserting the sensor into a body. The first duration is a duration in which a degree of stabilization of the sensor after sensor insertion is low, the second duration is a duration in which the degree of the stabilization of the sensor after a lapse of the first duration additionally progresses, and the third duration is a duration in which the degree of the stabilization of the sensor after a lapse of the second duration is higher than that in the second duration. The entire use time of the sensor may be variously divided into multiple calibration durations depending on a field to which the present disclosure is applied, which belongs to the scope of the present disclosure.

In operation S150, a calibration factor is calculated based on the calibration duration to which the time point at which the reference blood sugar value is input belongs. In operation S170, a calibrated blood sugar value is calculated by calibrating a measured blood sugar value by using the calculated calibration factor, and the calculated calibrated blood sugar value is provided to the user.

The calibration factor may be differently calculated depending on whether a modification condition of the calibration factor is satisfied at the time point at which the reference blood sugar value is input in the calibration duration to which the time point at which the reference blood sugar value is input belongs.

FIG. 6 is a diagram for describing an example of inputting calibration information according to a calibration period. Here, the calibration information is a reference blood sugar value of a user, which is measured with a test strip through an additional blood sugar meter.

Referring to FIG. 6, initial calibration information is input to a communication terminal at a time point $t_1$ at which a sensor of a sensor transmitter is initially stabilized. Here, the initial calibration information may be input multiple times in order to accurately calculate a calibration factor. The communication terminal calculates an initial calibration factor by using the initial calibration information and a blood sugar value measured in the sensor transmitter and calculates a calibrated blood sugar value of the user by calibrating the measured blood sugar value which is received from the sensor transmitter by using the initial calibration factor.

After the sensor of the sensor transmitter is initially stabilized, new calibration information is input to the communication terminal periodically, desirably, at twelve-hour or twenty-four-hour calibration periods or the like until a use period expiration time point of the sensor transmitter. After the sensor of the sensor transmitter is initially stabilized, the new calibration information may be input at intervals of a twelve-hour first calibration period $T_1$. Afterward, the sensor of the sensor transmitter may input the new calibration information at twenty-four-hour or forty-eight-hour second calibration periods $T_2$ or the like.

When the sensor is manufactured in an identical environment and under an identical condition, the sensor has a predetermined sensitivity drift characteristic after being inserted into a body. The calibration period may be differently set depending on a manufacturing environment for the sensor based on such a sensitivity drift characteristic.

FIG. 7 illustrates an example of a calibration duration dividing an entire use period of a sensor according to the present disclosure.

Referring to FIG. 7, a first duration $T_{is}$ is a duration with a low degree of stabilization of the sensor after initial stabilization. A second duration $T_{fs}$ is a duration in which stabilization information on the sensor after a lapse of the first duration is middle. The third duration $T_{es}$ is a duration with a degree of the stabilization of the sensor after a lapse of the second duration higher than that in the second duration.

The first duration may be set to 0.5 days to 1 day from a time point at which the sensor is inserted into a body. The second duration may be set to 3 days to 5 days from a time point at which at which the first duration is ended. The third duration may be set to a remaining use period of the sensor from a time point at which the second duration is ended. The first duration, the second duration, and the third duration may be set to times different from each other depending on a field to which the present disclosure is applied, which belongs to scope of the present disclosure.

FIG. 8 is a flowchart for describing an example of calculating a calibration factor in the present disclosure.

In specific description with reference to FIG. 8, in operation S171, whether a calibration duration to which a time point at which a reference blood sugar value is input belongs is a first duration is determined. When the calibration duration to which the time point at which the reference blood sugar value is input belongs is the first duration, in operation S179, a current calibration factor calculated from the reference blood sugar value and a measured blood sugar value corresponding to the time point at which the reference blood sugar value is input is calculated as a modified calibration factor.

Meanwhile, when the calibration duration to which the time point at which the reference blood sugar value is input belongs is not the first duration, in operation S173, whether the calibration duration to which the time point at which the reference blood sugar value is input belongs is a second duration is determined. When the calibration duration to which the time point at which the reference blood sugar value is input belongs is the second duration, in operation S175, whether a modification condition of the calibration factor is satisfied at the time point at which the reference blood sugar value is input is determined. When the calibration duration to which the time point at which the reference blood sugar value is input belongs is the second duration, and when a modification condition of a calibration condition at this point is not satisfied, in operation S179, the current calibration factor is calculated as the modified calibration factor. However, when the calibration duration to which the time point at which the reference blood sugar value is input belongs is the second duration, and when the modification condition of the calibration condition at this point is satisfied, a first weight value is granted to the current calibration factor in operation S176, and the current calibration factor is calculated in operation S179.

For example, when the calibration duration to which the time point at which the reference blood sugar value is input belongs is the second duration, and when the modification condition of the calibration condition at this point is satisfied, the modified calibration factor may be calculated as shown in the following Equation 1.

[Equation 1]

$$CF_R = \alpha_1 CF_C + (1 - \alpha_1) CF_P$$

Here, $CF_R$, $CF_C$, $CF_P$, and $\alpha_1$ denote the modified calibration factor, the current calibration factor, a previous calibration factor, and the first weight value, respectively.

Here, the first weight value may be set to 0.3 to 0.6. Here, the first weight value may be calculated in proportion to a difference value between a first biometric parameter and a second biometric parameter within a predetermined range of the first weight value.

Meanwhile, when the calibration duration to which the time point at which the reference blood sugar value is input belongs is not the second duration, in operation S 177, the calibration duration to which the time point at which the reference blood sugar value is input belongs is determined as a third duration, and whether the modification condition of the calibration factor is satisfied at the time point at which the reference blood sugar value is input is determined. When the calibration duration to which the time point at which the reference blood sugar value is input belongs is the third duration, and when the modification condition of the calibration factor at this point is not satisfied, in operation S179, the current calibration factor is calculated as the modified calibration factor. However, when the calibration duration to which the time point at which the reference blood sugar value is input belongs is the third duration, and when the modification condition of the calibration factor at this point is satisfied, a second weight value is granted to the current calibration factor in operation S178, and the current calibration factor is calculated in operation S179.

For example, when the calibration duration to which the time point at which the reference blood sugar value is input belongs is the third duration, and when a calibration parameter satisfies the modification condition of the condition at this point, the modified calibration factor may be calculated as shown in the following Equation 2.

[Equation 2]

$$CF_R = \alpha_2 CF_C + (1 - \alpha_2) CF_P$$

Here, $CF_R$, $CF_C$, $CF_P$, and $\alpha_2$ denote the modified calibration factor, the current calibration factor, the previous calibration factor, and the second weight value, respectively.

Here, the second weight value may be set to 0.6 to 0.9. Here, the second weight value may be calculated in proportion to the difference value between the first biometric parameter and the second biometric parameter within a predetermined range of the first weight value.

Here, the modification condition may be a condition for determining whether a biometric value of a user is unstable and may be set differently or identically depending on the calibration duration.

For example, an example of the modification condition is as follows.

1) a case in which while the reference blood sugar value exceeds an upper limit threshold blood sugar value, the current calibration factor is larger than the previous calibration factor
2) a case in which the reference blood sugar value is smaller than a lower limit threshold blood sugar value and the previous calibration factor is larger than the current calibration factor
3) a case in which the difference value between the first biometric parameter and the second biometric parameter exceeds a threshold value

In the present disclosure, the first biometric parameter may be a blood blood sugar value, and the second biometric parameter may be an interstitial fluid blood sugar value. The blood blood sugar value may be calculated through the above-described delay model between the blood blood sugar value and the interstitial fluid blood sugar value.

It may be identified that a difference between the blood blood sugar value and the interstitial fluid blood sugar value is not large in a state in which the biometric value of the user is stable, but the difference between the blood blood sugar value and the interstitial fluid blood sugar value is significant in a state in which the biometric value of the user is unstable. Thus, when the difference between the blood blood sugar value and the interstitial fluid blood sugar value exceeds a threshold value, the biometric value of the user may be determined as being in an unstable state.

FIG. 9 is a flowchart for describing an example of an operation of remodifying a modified calibration factor in the present disclosure.

When a calibration duration to which a time point at which a reference blood sugar value is input belongs is a third duration, since a degree of stabilization of a sensor is high, a calibration factor is required to be maintained so as not to be largely changed. To this end, the modified calibration factor may be remodified so as to follow an average value of calibration factors calculated in the third duration.

In specific description with reference to FIG. 9, an average value of previous modified calibration factors is calculated in the third duration from the time point at which the reference blood sugar value is input in operation S211, and a modification threshold range is calculated with the calculated average value as a reference in operation S213. Here, a previous modified calibration factor is a modified calibration factor previously acquired before the time point at which the reference blood sugar value is input.

In operation S215, whether the modified calibration factor is out of the modification threshold range is determined. In operation S217, a final calibration factor is determined by remodifying the modified calibration factor depending on whether the modified calibration factor is out of the modification threshold range.

In other words, when the modified calibration factor is within the modification threshold range, the modified calibration factor is determined to be the final calibration factor. However, when the modified calibration factor is out of the modification threshold range, in operation S219, the modified calibration factor is remodified to be in the modification threshold range, and the final calibration factor is determined. When the final calibration factor is determined, a calibrated blood sugar value is calculated by using the final calibration factor.

For example, when the modified calibration factor exceeds an upper limit of the modification threshold range, the modified calibration factor is remodified to be at the upper limit of the modification threshold range. When the modified calibration factor exceeds a lower limit of the modification threshold range, the modified calibration factor is remodified to be at the lower limit of the modification threshold range.

FIG. 10 is a flowchart for describing another example of an operation of remodifying a modified calibration factor in the present disclosure.

In specific description with reference to FIG. 10, an average value of previous modified calibration factors is calculated in a third duration from a time point at which a reference blood sugar value is input in operation S221, and a modification threshold range is calculated with the calculated average value as a reference in operation S223.

Here, a reward value may be calculated in consideration of a difference between the modified calibration factor and the average value so that the modified calibration factor follows the average value.

[Equation 4]

$$CF_{R2} = CF_R - (CF_R - CF_M) \times \alpha_3$$

Here, $CF_{R2}$, $CF_R$, $CF_M$, and $\alpha_3$ denote a final calibration factor, the modified calibration factor, the average value, and a third weight value, respectively. $(CF_R - CF_M) \times \alpha_3$ denotes the reward value.

Here, the third weight value may be set to 0.7 to 0.9.

For example, when the third weight value is set to 0.8, when the modified calibration factor is 10, and when the average value is 8, the final calibration factor may be remodified so as to follow the average value as shown in $CF_{R2}$=10-(10-8) $\times$ 0.8=8.4.

Meanwhile, the above-described example embodiments may be prepared with a computer-executable program and implemented in a general-purpose digital computer for operating the program with a computer-readable recording medium.

The computer-readable recording medium includes a magnetic storage medium (e.g., a read-only memory, a floppy disk, a hard disk, or the like), an optical reading medium (e.g., a compact disc (CD)-ROM, a digital versatile disc (DVD), or the like), and a storage medium such as a carrier wave (e.g., transmission through the Internet).

The present disclosure has been described with reference to example embodiments illustrated in the accompanying drawings, but it is merely an example. Those skilled in the art may understand that other various modifications and equivalents are possible therefrom. Thus, the true technical scope of the present disclosure should be determined by the technical idea of the accompanying claims.

## Claims

1. A calibration method for a biometric signal, the calibration method comprising:

    determining whether a reference biometric value is input;
    acquiring a first biometric parameter and a second biometric parameter at a time point at which the reference biometric value is input;
    determining whether a modification condition is satisfied based on the first biometric parameter and the second biometric parameter; and
    acquiring, with acquiring a modified calibration factor depending on whether the modification condition is satisfied, a calibrated biometric value from a biometric signal by using the modified calibration factor.

2. The calibration method of claim 1, wherein the acquiring of the calibrated biometric value from the biometric signal comprises, based on that the modification condition is not satisfied:

    calculating a current calibration factor from the input reference biometric value; and
    acquiring the calibrated biometric value from the biometric signal based on the current calibration factor.

3. The calibration method of claim 1, wherein the acquiring of the calibrated biometric value from the biometric signal comprises, based on the modification condition being satisfied:

    acquiring a current calibration factor from the input reference biometric value;
    determining a calibration duration to which the time point at which the reference biometric value is input belongs;
    acquiring the modified calibration factor from the current calibration factor based on the determined calibration duration;
    measuring the biometric signal by using a sensor;
    acquiring an interstitial fluid calibrated biometric value based on the biometric signal and the modified calibration factor; and
    acquiring a blood calibrated biometric value based on the interstitial fluid calibrated biometric value.

4. The calibration method of claim 3, wherein the calibration duration is divided into a first duration, a second duration after the first duration, and a third duration after the second duration.

5. The calibration method of claim 4, wherein based on that the time point at which the reference biometric value is input belongs to the first duration, the modified calibration factor is the current calibration factor.

6. The calibration method of claim 4, wherein based on that the time point at which the reference biometric value is input belongs to the second duration, the acquiring of the modified calibration factor from the current calibration factor comprises applying a first weight value to the current calibration factor.

7. The calibration method of claim 4, wherein based on that the time point at which the reference biometric value is input belongs to the third duration, the acquiring of the modified calibration factor from the current calibration factor comprises applying a second weight value to the current calibration factor.

8. The calibration method of any one of claims 1 through 7, wherein the first biometric parameter is an interstitial fluid (ISF) blood sugar value, and
the second biometric parameter is a blood or plasma blood sugar value.

**9.** The calibration method of claim 4, further comprising, based on that the time point at which the reference biometric value is input belongs to the third duration:

acquiring at least one or more previous modified calibration factors;
acquiring an average value of the previous modified calibration factors; and
remodifying the modified calibration factor based on the average value.

**10.** The calibration method of claim 9, wherein the remodifying of the modified calibration factor comprises:

acquiring a modification threshold range based on the average value;
determining whether the modified calibration factor is out of the modification threshold range; and
remodifying the modified calibration factor depending on whether the modified calibration factor is out of the modification threshold range, and
the calibration method further comprises:

acquiring a final calibration factor based on the remodified modified calibration factor; and
calculating the calibrated biometric value based on the final calibration factor.

**11.** The calibration method of claim 10, based on that the modified calibration factor is out of the modification threshold range, further comprising remodifying the modified calibration factor so that the modified calibration factor belongs to the modification threshold range.

**12.** The calibration method of claim 10, wherein based on that the modified calibration factor is within the modification threshold range, the modified calibration factor is the final calibration factor.

**13.** The calibration method of claim 9, wherein the remodifying of the modified calibration factor comprises:

acquiring a difference between the modified calibration factor and the average value;
acquiring a reward value based on the difference between the modified calibration factor and the average value; and
remodifying the modified calibration factor based the reward value, and
wherein the calibration method further comprises:

acquiring a final calibration factor based on the remodified modified calibration factor; and
acquiring the calibrated biometric value based on the final calibration factor.

FIG. 1

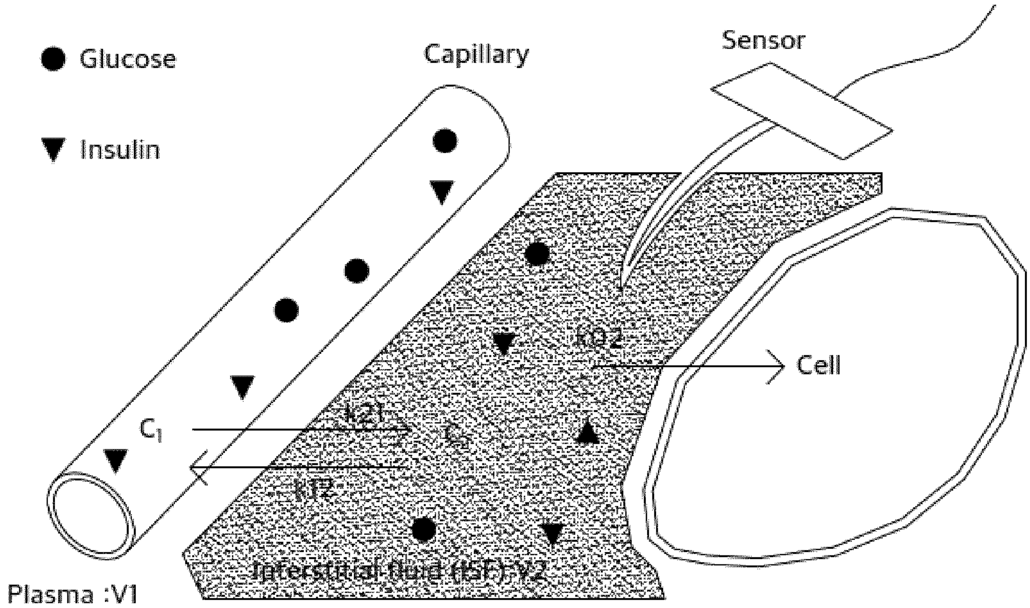
Glucose
Insulin
Capillary
Sensor
Cell
k21
k12
k02
$C_1$
$C_2$
Interstitial fluid (ISF) :V2
Plasma :V1

FIG. 2

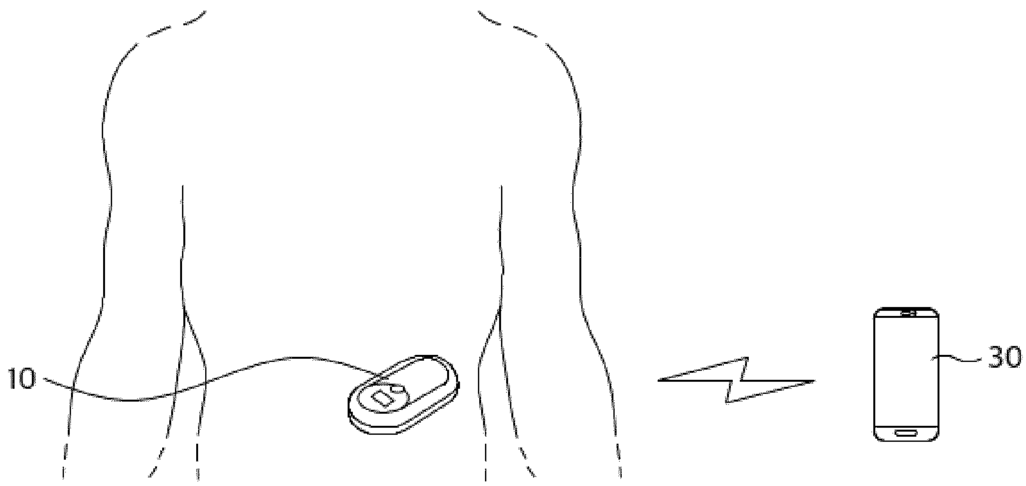
10
30

FIG. 3

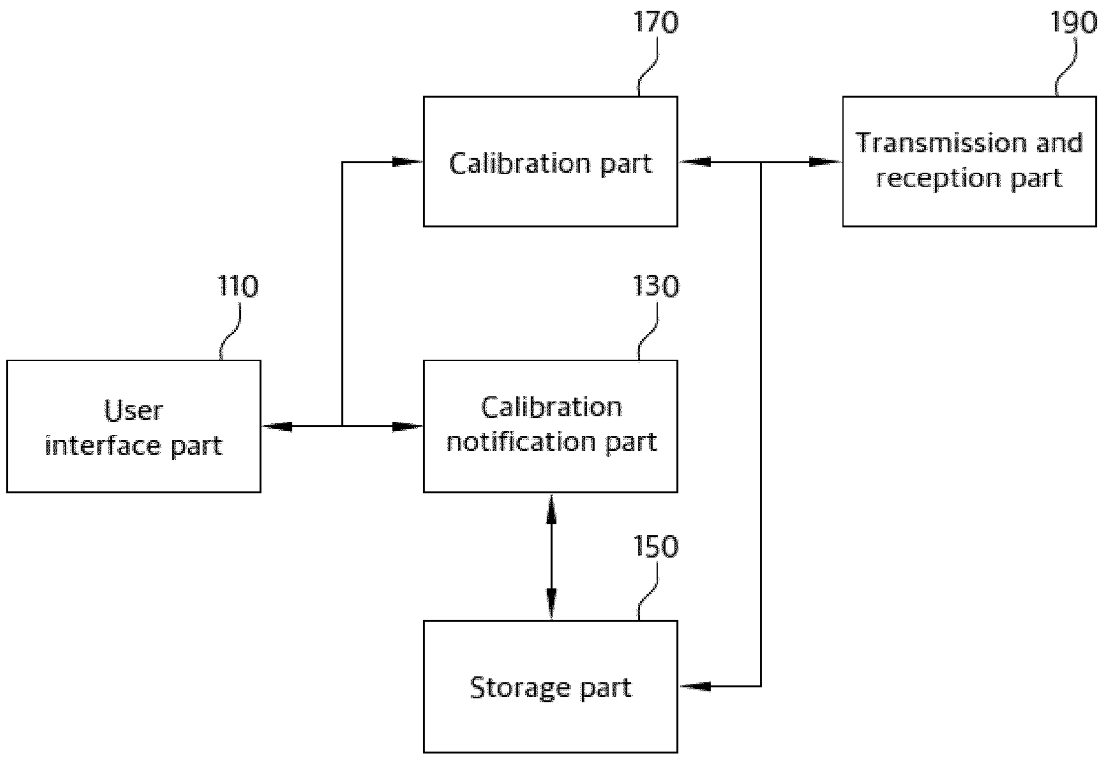
170
190
Calibration part
Transmission and reception part
110
130
User interface part
Calibration notification part
150
Storage part

FIG. 4

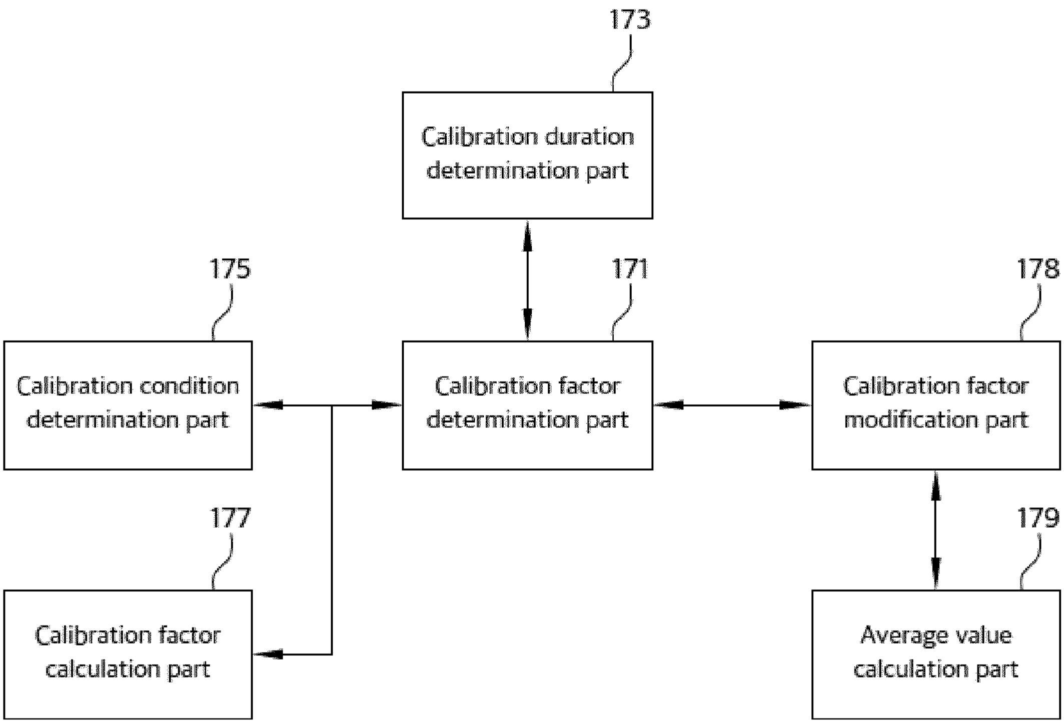
173
Calibration duration determination part
175
171
178
Calibration condition determination part
Calibration factor determination part
Calibration factor modification part
177
179
Calibration factor calculation part
Average value calculation part

FIG. 5

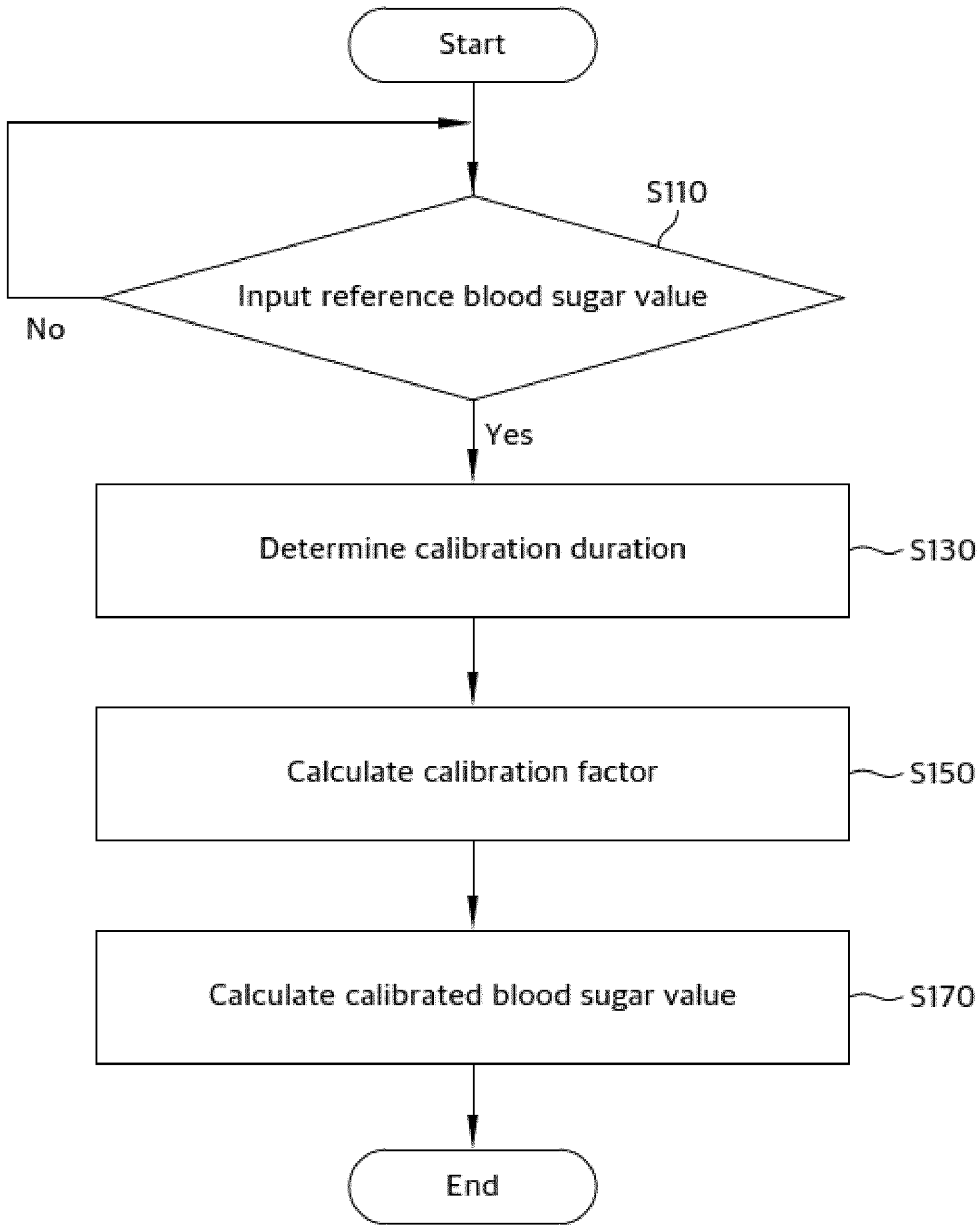
Start
S110
Input reference blood sugar value
No
Yes
Determine calibration duration
S130
Calculate calibration factor
S150
Calculate calibrated blood sugar value
S170
End

FIG. 6

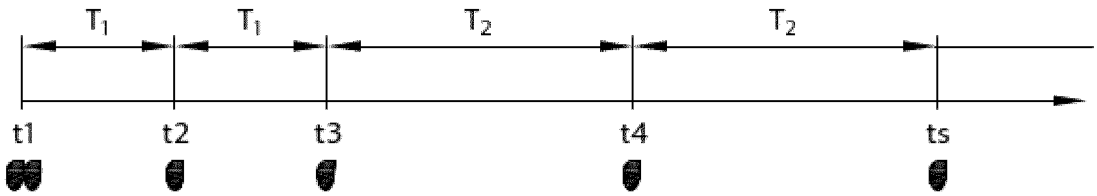


FIG. 7

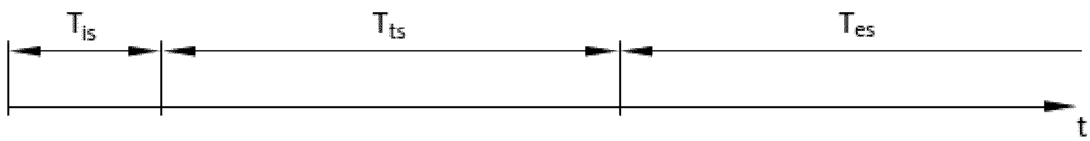

FIG. 8

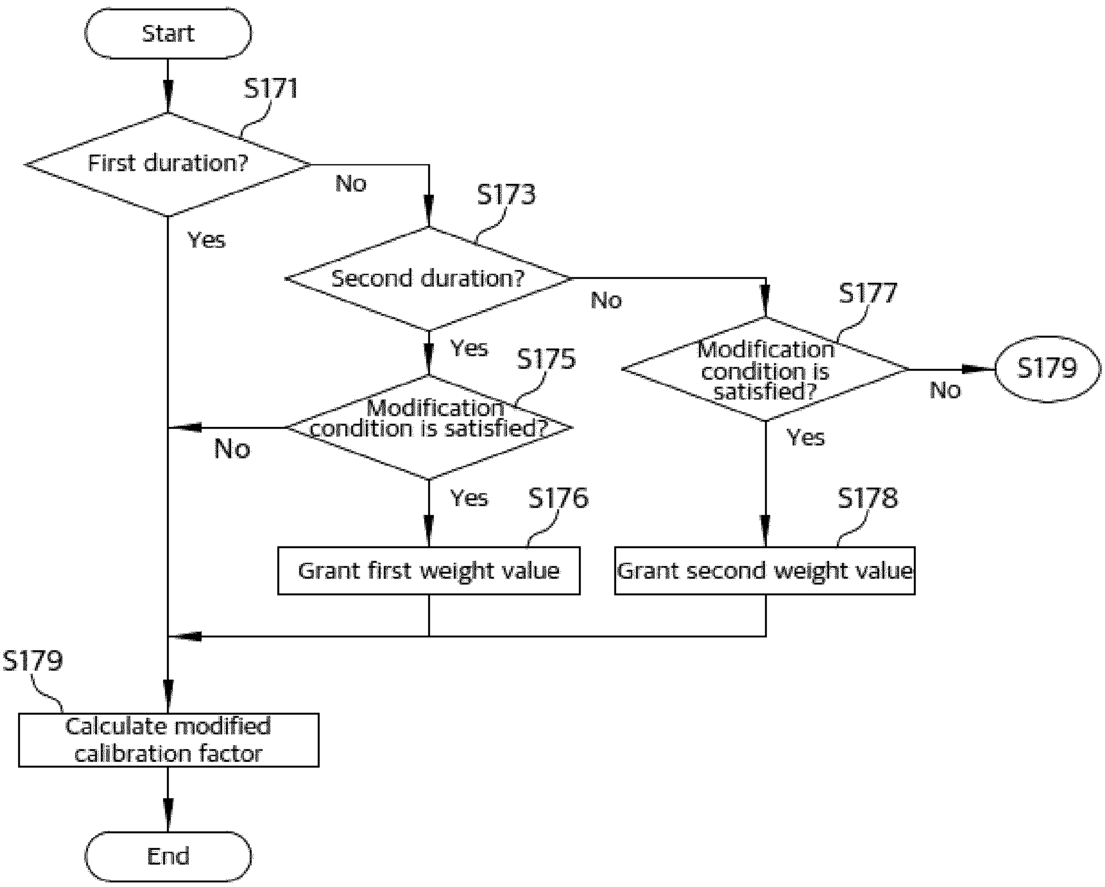
Start
S171
First duration?
No
Yes
S173
Second duration?
No
Yes
S177
Modification condition is satisfied?
No
S179
Yes
S175
Modification condition is satisfied?
No
Yes
S176
Grant first weight value
S178
Grant second weight value
S179
Calculate modified calibration factor
End

FIG. 9

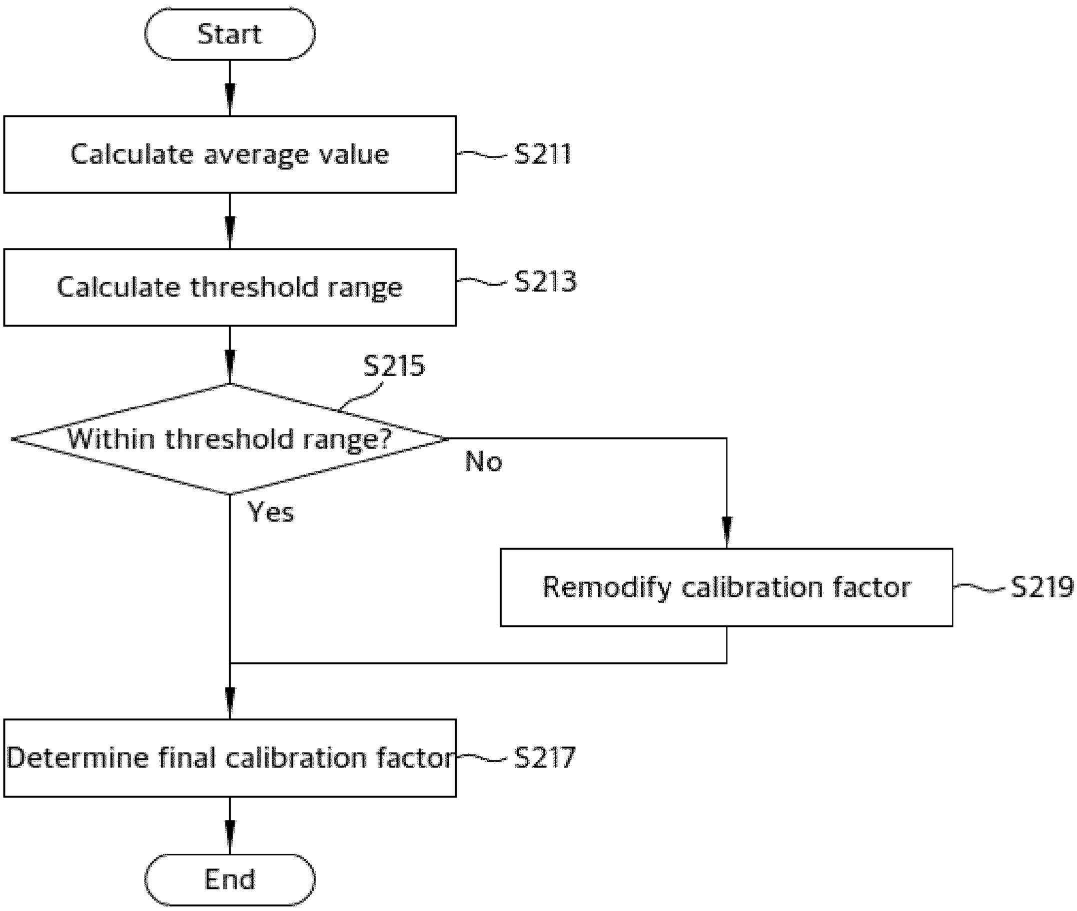
Start
Calculate average value
S211
Calculate threshold range
S213
S215
Within threshold range?
No
Yes
Remodify calibration factor
S219
Determine final calibration factor
S217
End

FIG. 10

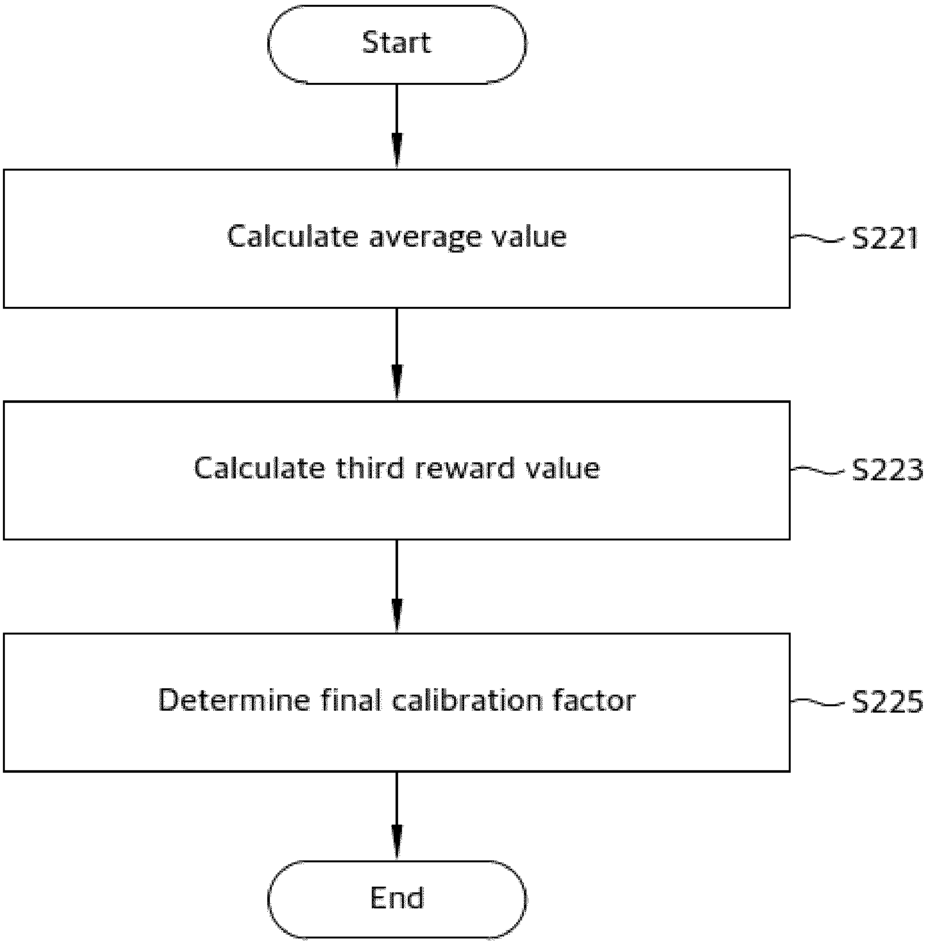
Start
Calculate average value
S221
Calculate third reward value
S223
Determine final calibration factor
S225
End

# INTERNATIONAL SEARCH REPORT

International application No.
**PCT/KR2023/000398**

**A. CLASSIFICATION OF SUBJECT MATTER**

**A61B 5/1495**(2006.01)i; **A61B 5/145**(2006.01)i

According to International Patent Classification (IPC) or to both national classification and IPC

**B. FIELDS SEARCHED**

Minimum documentation searched (classification system followed by classification symbols)

A61B 5/1495(2006.01); A61B 5/00(2006.01); A61B 5/021(2006.01); A61B 5/05(2006.01); A61B 5/145(2006.01); A61B 5/1455(2006.01); G01N 21/35(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 기준 (standard), 생체 (physiology), 파라미터 (parameter), 교정 (correct), 체액 (body fluid), 혈당 (blood glucose)

**C. DOCUMENTS CONSIDERED TO BE RELEVANT**

| Category* | Citation of document, with indication, where appropriate, of the relevant passages | Relevant to claim No. |
|---|---|---|
| Y<br>A | EP 2840958 B1 (ABBOTT DIABETES CARE, INC.) 30 September 2020 (2020-09-30)<br>See paragraph [0112]; and claim 1. | 1,2,8<br>3-7,9-13 |
| Y | KR 10-2035424 B1 (UNIST(ULSAN NATIONAL INSTITUTE OF SCIENCE AND TECHNOLOGY)) 22 October 2019 (2019-10-22)<br>See paragraphs [0055], [0057] and [0063]. | 1,2,8 |
| A | KR 10-2021-0064651 A (I-SENS, INC.) 03 June 2021 (2021-06-03)<br>See entire document. | 1-13 |
| A | JP 2011-062335 A (PANASONIC ELECTRIC WORKS CO., LTD.) 31 March 2011 (2011-03-31)<br>See entire document. | 1-13 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 April 2023** | **11 April 2023** |
| Name and mailing address of the ISA/KR<br>**Korean Intellectual Property Office**<br>**Government Complex-Daejeon Building 4, 189 Cheongsa-ro, Seo-gu, Daejeon 35208** | Authorized officer |
| Facsimile No. **+82-42-481-8578** | Telephone No. |

Form PCT/ISA/210 (second sheet) (July 2022)

INTERNATIONAL SEARCH REPORT

International application No.
**PCT/KR2023/000398**

**C. DOCUMENTS CONSIDERED TO BE RELEVANT**

| Category* | Citation of document, with indication, where appropriate, of the relevant passages | Relevant to claim No. |
|---|---|---|
| A | KR 10-2020-0054719 A (SAMSUNG ELECTRONICS CO., LTD.) 20 May 2020 (2020-05-20) See entire document. | 1-13 |

Form PCT/ISA/210 (second sheet) (July 2022)

**INTERNATIONAL SEARCH REPORT**
**Information on patent family members**

International application No.
**PCT/KR2023/000398**

| Patent document cited in search report | Publication date (day/month/year) | Patent family member(s) | Publication date (day/month/year) |
|---|---|---|---|
| EP 2840958 B1 | 30 September 2020 | EP 2840958 A1 | 04 March 2015 |
| | | EP 3777664 A1 | 17 February 2021 |
| | | US 10561372 B2 | 18 February 2020 |
| | | US 2013-0304389 A1 | 14 November 2013 |
| | | US 2017-0095213 A1 | 06 April 2017 |
| | | US 2020-0146634 A1 | 14 May 2020 |
| | | US 9462970 B2 | 11 October 2016 |
| | | WO 2013-163342 A1 | 31 October 2013 |
| KR 10-2035424 B1 | 22 October 2019 | None | |
| KR 10-2021-0064651 A | 03 June 2021 | AU 2020-393447 A1 | 14 October 2021 |
| | | AU 2020-393447 B2 | 13 October 2022 |
| | | EP 3932310 A1 | 05 January 2022 |
| | | JP 2022-530511 A | 29 June 2022 |
| | | JP 7247370 B2 | 28 March 2023 |
| | | KR 10-2022-0130646 A | 27 September 2022 |
| | | US 2022-0160266 A1 | 26 May 2022 |
| | | WO 2021-107335 A1 | 03 June 2021 |
| JP 2011-062335 A | 31 March 2011 | None | |
| KR 10-2020-0054719 A | 20 May 2020 | US 11219375 B2 | 11 January 2022 |
| | | US 2020-0146563 A1 | 14 May 2020 |

Form PCT/ISA/210 (patent family annex) (July 2022)